(19) Europäisches Patentamt
European Patent Office
Office européen des brevets

(11) Numéro de publication : **0 545 805 A1**

(12) **DEMANDE DE BREVET EUROPEEN**

(21) Numéro de dépôt : **92403233.7**

(51) Int. Cl.$^5$ : **A61K 31/135,** A61K 31/235

(22) Date de dépôt : **01.12.92**

(30) Priorité : **06.12.91 FR 9115122**

(43) Date de publication de la demande :
**09.06.93 Bulletin 93/23**

(84) Etats contractants désignés :
**AT BE CH DE DK ES FR GB GR IE IT LI LU NL PT SE**

(71) Demandeur : **ADIR ET COMPAGNIE**
**1 rue Carle Hébert**
**F-92415 Courbevoie Cédex (FR)**

(72) Inventeur : **Wierzbicki, Michel**
**8 Résidence du Chemin Pavé**
**F-78620 L Etang la Ville (FR)**
Inventeur : **Hugon, Pierre**
**13 rue Eugène Sue**
**F-92500 Rueil Malmaison (FR)**
Inventeur : **Duhault, Jacques**
**14bis rue Paul Demange**
**F-78290 Croissy sur Seine (FR)**
Inventeur : **Espinal, Joseph**
**11 rue Chateaubriand**
**F-75008 Paris (FR)**

(74) Mandataire : **Reverbori, Marcelle**
**Adir et Compagnie, 1, rue Carle Hébert**
**F-92415 Courbevoie Cédex (FR)**

(54) **Utilisation de la D-fenfluramine et de dérivés de la fenfluramine dans le traitement de l'hypertension chez des sujets insulino-résistants.**

(57) Utilisation pour l'obtention de médicaments destinés au traitement de l'hypertension chez des sujets insulino-résistants,
— de dérivés de formule :

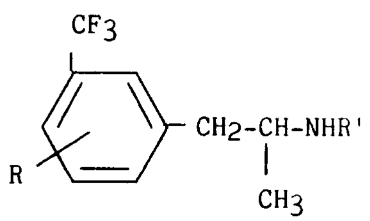

(I)

dans laquelle :
— R est hydrogène ou halogène, et
— R′ est :
— un radical hydrocarboné de $C_1$ à $C_5$ saturé ou non, ou : un des radicaux :
$-CH_2COOR_1$,

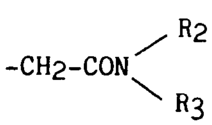

,

$(-CH_2)_nOR_4$ et $-COOR_5$ ;
($R_1$, $R_2$, $R_3$, $R_4$, $R_5$, et n étant tels que définis dans la description),
— sous forme dextrogyre lorsque R′ est éthyl, et sous forme racémique et optiquement active dans tous les autres cas,
— ainsi que leurs sels physiologiquement tolérables.

EP 0 545 805 A1

Jouve, 18, rue Saint-Denis, 75001 PARIS

La présente invention concerne l'utilisation de la d-fenfluramine et de dérivés de la fenfluramine pour l'obtention de médicaments destinés au traitement de l'hypertension chez des sujets présentant une insulino-résistance associée ou non à d'autres anomalies métaboliques.

Plus spécifiquement, l'invention concerne l'utilisation, pour l'obtention de médicaments destinés au traitement de l'hypertension chez des sujets présentant une insulino-résistance associée ou non à d'autres anomalies métaboliques, les dérivés de formule générale I :

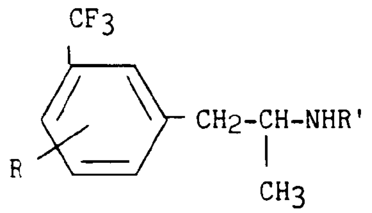

(I)

dans laquelle :

- R représente un atome d'hydrogène ou un atome d'halogène tel que par exemple, un atome de chlore, brome ou fluor ;
- R' représente :
  1) un radical hydrocarboné renfermant de 1 à 5 atomes de carbone, en chaine droite ou ramifiée, contenant éventuellement une double ou une triple liaison,
  2) un radical de formule :

$$-CH_2COOR_1$$

  dans laquelle $R_1$ représente :
    a) un atome d'hydrogène,
    b) un radical alkyle en chaine droite ou ramifiée ayant de 1 à 5 atomes de carbone,
    c) un radical cycloalkyle ayant de 3 à 7 atomes de carbone,
    d) un radical benzyle, halobenzyle, ($C_{1-5}$ alkyl)-benzyle, ($C_{1-5}$ alkoxy)-benzyle ou méthylènedioxybenzyle, ou
    e) un radical de formule :

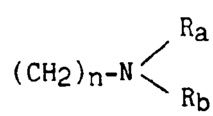

    dans laquelle :
    - n représente 2 ou 3 et
    - $R_a$ et $R_b$ identiques ou différents représentent chacun un radical alkyle en chaine droite ou ramifiée ayant de 1 à 5 atomes de carbone, ou
      $R_a$ et $R_b$ forment ensemble avec l'atome d'azote auquel ils sont liés un radical pyrrolidinyle, pipéridyle, morpholino, thiomorpholino, azabicyclo (3,3,0) octyle ou azabicyclo (3,2,2) nonyle,
  3) un radical de formule :

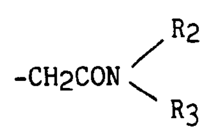

  dans laquelle :
  $R_2$ et $R_3$, identiques ou différents, représentent chacun :
    a) un atome d'hydrogène,
    b) un radical alkyle de 1 à 5 atomes de carbone en chaine droite ou ramifiée,
    c) un radical phényle ou un radical pyridyle chacun éventuellement substitué par un ou plusieurs atomes d'halogène ou radicaux alkyle ou alkoxy ayant chacun de 1 à 4 atomes de carbone ou par un radical trifluorométhyle ou
    d) $R_2$ et $R_3$ forment ensemble avec l'atome d'azote auquel ils sont liés un radical hétérocyclique choisi parmi les radicaux : morpholino, pyrrolidinyle, pipéridino, indolinyle et isoindolinyle, et

e) lorsque $R_2$ représente un atome d'hydrogène, $R_3$ représente, de plus :

- soit un radical $-CONH_2$
- soit un radical

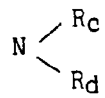

dans lequel $R_c$ et $R_d$ identiques ou différents représentent chacun un atome d'hydrogène ou un radical alkyle de 1 à 4 atomes de carbone en chaine droite ou ramifiée,

4) un radical de formule :

$$-(CH_2)_n-OR_4$$

dans laquelle :

- n représente 2 ou 3, et
- $R_4$ représente :

a) un atome d'hydrogène, ou

b) un radical de formule :

$$-COR'_4$$

dans laquelle $R'_4$ représente :

α) un radical hydrocarboné en chaine droite ou ramifiée ayant de 1 à 5 atomes de carbone contenant éventuellement une double ou une triple liaison, et/ou éventuellement substitué par un ou plusieurs atomes d'halogène, ou radicaux hydroxy, $(C_{1-3})$alcoxy ou carboxyle,

β) un radical cycloalkyle contenant de 3 à 7 atomes de carbone,

ν) un radical phényle ou naphtyle chacun éventuellement substitué par un ou plusieurs radicaux alkyl ou alkoxy ayant chacun de 1 à 5 atomes de carbone, nitro, amino, trifluorométhyle ou méthylènedioxy,

δ) un radical benzyle, phénéthyle ou cinnamyle, chacun éventuellement substitué sur le noyau phényle par un ou plusieurs atomes d'halogène ou radicaux alkyle ou alkoxy ayant chacun de 1 à 5 atomes de carbone, nitro, amino, trifluorométhyle et méthylènedioxy, ou

ε) un radical phénoxy $(C_{1-4})$ alkyle, éventuellement substitué sur le noyau phényle par un ou plusieurs atomes d'halogène, ou

5) un radical de formule :

$$-COOR_5$$

dans laquelle :

$R_5$ représente un radical alkyle contenant de 1 à 4 atomes de carbone ;

ces dérivés de formule I étant sous forme racémique ou optiquement active sauf lorsque R' représente un radical éthyle où seul l'isomère dextrogyre est concerné,

et leurs sels physiologiquement tolérables.

Les dérivés de formule (I) et leurs sels ont notamment fait l'objet des brevets français n° 1.324.220, 1.461.162, 1.517.587, 1.570.822, 2.029.385, 2.034.571, 2.240.726, 2.269.726, et 6.626 M.

Il est d'autre part connu que certains produits apparentés aux dérivés (I), dont par exemple la dl fenfluramine, sont capables de réduire l'insulino-résistance et l'hyperinsulinémie basale, cf : Storlien et al. Diabetes (1989), 38, 499-503 ; Brindley et al. Biochim. Biophys. Acta (1991), 1085, 119-125 ; Carr et al. Proc. Soc. Exp. Biol. Med. (1977), 154, 116-120 ; Duhault et al. Atherosclerosis (1976), 23, 63-72 ; Pestell et al. Diabetes Care (1989), 12, 252-258.

La demanderesse a présentement découvert que les dérivés de formule I et leurs sels physiologiquement tolérables, bien que dépourvus d'activité hypotensive intrinsèque, diminuent la pression artérielle des sujets insulino-résistants et de ce fait peuvent être utilisés en thérapeutique dans le traitement de l'hypertension associée aux états d'insulino-résistance et autres anomalies métaboliques telles que par exemple obésité, dyslipémie, hyperinsulinémie etc... qui constituent des facteurs de risques cardiovasculaires importants (coronaropathies, macroangiopathie, etc...).

La présente invention a également pour objet les compositions pharmaceutiques contenant comme principe actif un dérivé de formule I ou un de ses sels physiologiquement tolérables, mélangé ou associé à un excipient pharmaceutique approprié.

Les compositions pharmaceutiques ainsi obtenues sont présentées avantageusement sous des formes convenant pour l'administration par voie orale, ou parentérale telles que par exemple comprimés, dragées, gelules, solutions injectables ou buvables.

La posologie varie selon l'âge et le poids du patient, la voie d'administration, la nature des traitements associés et s'échelonne de 15 à 150 mg de principe actif par prise, 1 à 3 fois par jour.

L'étude pharmacologique suivante illustre la présente invention sans toutefois la limiter.

## ETUDE PHARMACOLOGIQUE

### 1- Etude sur le rat SHR

Cette souche de rat présente une hypertension génétique. L'administration de 3 mg/kg P.O. de d-fenfluramine diminue la pression artérielle de $9.10^2$ Pa chez ces animaux.

### 2- Etude du traitement chronique du rat âgé

Au cours du vieillissement, une insulino-résistance apparaît, accompagnée d'une hyperinsulinémie et d'une élévation de la pression artérielle (cf tableau 1 ci-après).

**Tableau 1**

| | Insulinémie µU/ml | Pression artérielle $10^2$ Pa |
|---|---|---|
| **Rats 3 mois** | 16 ± 1 | 133 ± 9 |
| **Rats 12 mois** | 32 ± 3 | 170 ± 8 |

Le traitement par la d-fenfluramine à la dose de 2,5 mg/kg 2 fois par jour pendant 3 semaines, diminue la pression artérielle du rat âgé de $12.10^2$ Pa sans modifier celle du rat jeune.

Cet effet est relié à une normalisation de la réactivité vasculaire, en particulier à la sérotonine et à l'ADP (cf tableau 2 ci-après).

**Tableau 2**

| | Maximum de contraction | |
|---|---|---|
| | Sérotonine* | ADP** |
| Rats 3 mois | 181 | 54 |
| Rats 12 mois | 213 | 88 |
| Rats 12 mois (traités d-fenfluramine) | 145 | 66 |

exprimé par rapport à KCl* ou la phényléphrine** auxquels on attribue une contraction de 100 %.

Des résultats analogues sont obtenus avec d'autres produits de l'invention tels que par exemple le benfluorex à la dose de 50 mg/kg, 5 fois par jour.

3- Conclusion

Les études ci-dessus montrent l'intérêt de l'utilisation des dérivés I et tout principalement de la d-fenfluramine et du benfluorex pour l'obtention de médicaments destinés au traitement de l'hypertension chez des sujets présentant une insulino-résistance associée ou non à d'autres anomalies métaboliques.

## Revendications

**1-** Utilisation, pour l'obtention de médicaments destinés au traitement de l'hypertension chez des sujets insulino-résistants, des dérivés de formule générale I :

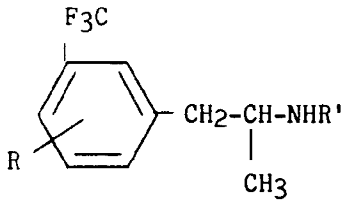

(I)

dans laquelle :

- R représente un atome d'hydrogène ou un atome d'halogène ;
- R' représente :
  1) un radical hydrocarboné renfermant de 1 à 5 atomes de carbone, en chaine droite ou ramifiée, contenant éventuellement une double ou une triple liaison,
  2) un radical de formule :

$$-CH_2COOR_1$$

  dans laquelle $R_1$ représente :
    a) un atome d'hydrogène,
    b) un radical alkyle en chaine droite ou ramifiée ayant de 1 à 5 atomes de carbone,
    c) un radical cycloalkyle ayant de 3 à 7 atomes de carbone,
    d) un radical benzyle, halobenzyle, ($C_{1-5}$ alkyl)-benzyle, ($C_{1-5}$ alkoxy)-benzyle ou méthylènedioxybenzyle, ou
    e) un radical de formule :

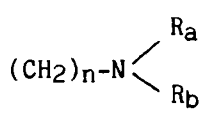

    dans laquelle :
    - n représente 2 ou 3 et
    - $R_a$ et $R_b$ identiques ou différents représentent chacun un radical alkyle en chaine droite ou ramifiée ayant de 1 à 5 atomes de carbone, ou
      $R_a$ et $R_b$ forment ensemble avec l'atome d'azote auquel ils sont liés un radical pyrrolidinyle, pipéridyle, morpholino, thiomorpholino, azabicyclo (3,3,0) octyle ou azabicyclo (3,2,2) nonyle,

  3) un radical de formule :

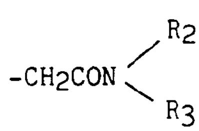

  dans laquelle :

$R_2$ et $R_3$, identiques ou différents, représentent chacun :

a) un atome d'hydrogène,

b) un radical alkyle de 1 à 5 atomes de carbone en chaine droite ou ramifiée,

c) un radical phényle ou un radical pyridyle chacun éventuellement substitué par un ou plusieurs atomes d'halogène ou radicaux alkyle ou alkoxy ayant chacun de 1 à 4 atomes de carbone ou par un radical trifluorométhyle ou

d) $R_2$ et $R_3$ forment ensemble avec l'atome d'azote auquel ils sont liés un radical hétérocyclique choisi parmi les radicaux : morpholino, pyrrolidinyle, pipéridino, indolinyle et isoindolinyle, et

e) lorsque $R_2$ représente un atome d'hydrogène, $R_3$ représente, de plus :

- soit un radical $-CONH_2$
- soit un radical

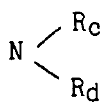

dans lequel $R_c$ et $R_d$ identiques ou différents représentent chacun un atome d'hydrogène ou un radical alkyle de 1 à 4 atomes de carbone en chaine droite ou ramifiée,

4) un radical de formule :

$$-(CH_2)_n-OR_4$$

dans laquelle :

- n représente 2 ou 3, et
- $R_4$ représente :

a) un atome d'hydrogène, ou

b) un radical de formule :

$$-COR'_4$$

dans laquelle R'4 représente :

α) un radical hydrocarboné en chaine droite ou ramifiée de 1 à 5 atomes de carbone contenant éventuellement une double ou une triple liaison, et/ou éventuellement substitué par un ou plusieurs atomes d'halogène, ou radicaux hydroxy, $(C_{1-3})$alcoxy ou carboxyle,

β) un radical cycloalkyle contenant de 3 à 7 atomes de carbone,

ν) un radical phényle ou naphtyle chacun éventuellement substitué par un ou plusieurs radicaux alkyl ou alkoxy ayant chacun de 1 à 5 atomes de carbone, nitro, amino, trifluorométhyle ou méthylènedioxy,

δ) un radical benzyle, phénéthyle ou cinnamyle, chacun éventuellement substitué sur le noyau phényle par un ou plusieurs atomes d'halogène ou radicaux alkyle ou alkoxy ayant chacun de 1 à 5 atomes de carbone, nitro, amino, trifluorométhyle et méthylènedioxy, ou

ε) un radical phénoxy $(C_{1-4})$ alkyle, éventuellement substitué sur le noyau phényle par un ou plusieurs atomes d'halogène, ou

5) un radical de formule :

$$-COOR_5$$

dans laquelle :

$R_5$ représente un radical alkyle contenant de 1 à 4 atomes de carbone ;

sous forme d'isomère dextrogyre dans le cas où R' représente un radical éthyle et sous forme racémique et optiquement active dans tous les autres cas,

ainsi que leurs sels physiologiquement tolérables.

**2-** Utilisation, selon la revendication 1, pour l'obtention de médicaments destinés au traitement de l'hypertension chez des sujets insulino-résistants, de la d-fenfluramine.

**3-** Utilisation, selon la revendication 1, pour l'obtention de médicaments destinés au traitement de l'hypertension chez des sujets insulino-résistants, du benfluorex.

**4-** Les compositions pharmaceutiques pour le traitement de l'hypertension chez des sujets insulino-résistants, renfermant comme principe actif au moins un dérivé de formule I définie dans la revendication 1 ou un de ses sels d'addition physiologiquement tolérables.

**5-** Les compositions pharmaceutiques pour le traitement de l'hypertension chez des sujets insulino-résistants, renfermant comme principe actif la d-fenfluramine.

**6-** Les compositions pharmaceutiques pour le traitement de l'hypertension chez des sujets insulino-résistants, renfermant comme principe actif le benfluorex.

**7-** Les compositions pharmaceutiques, selon les revendications 4 à 6, caractérisées en ce qu'elles renferment de 15 à 150 mg de principe actif, mélangé ou associé à un excipient pharmaceutique approprié.

Office européen des brevets

# RAPPORT DE RECHERCHE EUROPEENNE

Numero de la demande

EP 92 40 3233

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,P | LA PRESSE MEDICALE, vol. 21, no. 28, 9 septembre 1992, pages 1330-1335, Paris, FR; D.N. BRINDLEY: "Mode d'action du benfluorex" * Pages 1330,1334 * | 1-7 | A 61 K 31/135<br>A 61 K 31/235 |
| X | DIABETES, vol. 38, avril 1989, pages 499-503; L.H. STORLIEN et al.: "Effect of d-fenfluramine on basal glucose turnover and fat-feeding-induced insulin resistance in rats" * Abrégé; page 500, colonne de gauche, lignes 5-10 * | 4-7 | |
| Y | IDEM | 1-3 | |
| Y | BIOCHIMICA ET BIOPHYSICA ACTA, vol. 1085, août 1991, pages 119-125, Elsevier Science Publishers B.V.; D.N. BRINDLEY et al.: "Decreased serum lipids, serum insulin and triacylglycerol synthesis in adipose tissue of JCR:LA-corpulent rats treated with benfluorex" * Abrégé; page 120, colonne de gauche, lignes 13-19,36-40 * | 1-3 | **DOMAINES TECHNIQUES RECHERCHES (Int. Cl.5)**<br>A 61 K |
| Y | ATHEROSCLEROSIS, vol. 23, 1976, pages 63-72, Elsevier Scientific Publishing Co., Amsterdam, NL; J. DUHAULT et al.: "780 SE: a new type of hypolipemic agent comparative assays in rats" * Abrégé; page 69, lignes 16-18 * --- -/- | 1-3 | |

Le présent rapport a été établi pour toutes les revendications

| Lieu de la recherche | Date d'achèvement de la recherche | Examinateur |
|---|---|---|
| LA HAYE | 05-03-1993 | GERLI P F M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Page 2

Office européen des brevets

# RAPPORT DE RECHERCHE EUROPEENNE

Numero de la demande

EP 92 40 3233

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | JOURNAL OF THE ROYAL COLLEGE OF GENERAL PRACTITIONERS, vol. 27, no. 181, août 1977, pages 497-501; K.D. HUDSON: "The anorectic and hypotensive effect of fenfluramine in obesity" * Page 499, colonne de droite, lignes 1-8; page 501, colonne de gauche, lignes 5-7 * --- | 1-3 | |
| Y | ULSTER MED. J., vol. 44, no. 1, 1975, pages 55-61; M.E. SCOTT et al.: "Fenfluramine in the treatment of hypertensive patients with refractory obesity" * Page 60, lignes 15-21 * --- | 1-3 | |
| Y | POSTGRAD. MEDICAL JOURNAL, vol. 51 (suppl. 1), 1975, page 158; B.K. ADADEVOH: "Therapeutic effect of fenfluramine in obese Nigerians-clinical, metabolic and hypotensive properties" * Le document en entier * --- | 1-3 | DOMAINES TECHNIQUES RECHERCHES (Int. Cl.5) |
| A | DIALOG INFORMATION SERVICES, file 155: MEDLINE, accession no. 05251988; D.H. LOCKWOOD et al.: "Cellular alterations responsible for insulin resistance in obesity and type II diabetes mellitus", & AM. J. MED. 1983, 75(5B), P23-31 --- -/- | 1-7 | |

Le présent rapport a été établi pour toutes les revendications

| Lieu de la recherche | Date d'achèvement de la recherche | Examinateur |
|---|---|---|
| LA HAYE | 05-03-1993 | GERLI P F M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)

Page 3

Office européen des brevets

# RAPPORT DE RECHERCHE EUROPEENNE

Numero de la demande

EP 92 40 3233

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DIABETES CARE, vol. 13, suppl. 3, août 1990, pages 53-58; A. MELANDER et al.: "Will sulfonylurea treatment of impaired glucose tolerance delay development and complications of NIDDM?" * Page 54 * ----- | | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 05-03-1993 | GERLI P F M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
&: membre de la même famille, document correspondant

EPO FORM 1503 03.82 (P0402)